# FASCICULE DE BREVET EUROPEEN

(11) **EP 1 303 296 B1**
(45) Date de publication et mention de la délivrance du brevet: **13.04.2005**
(21) Numéro de dépôt: 01958190.9
(22) Date de dépôt: 27.07.2001
(51) Int. Cl.: A61K 38/40, A61P 7/06

(54) **COMPOSITION CONTENANT UNE PROTEINE COMPLEXANT LE FER ET UN PRECURSEUR DU METABOLISME DU MONOXYDE D'AZOTE ET/OU UN DONNEUR CHIMIQUE DE MONOXYDE D'AZOTE ET UTILISATIONS**
ZUSAMMENSETZUNG, WELCHE EIN EISENKOMPLEXIERENDES PROTEIN UND EINEN VORLÄUFER IM STOFFWECHSEL VON STICKSTOFFOXID ODER EINEN STICKSTOFFOXID DONOR ENTHÄLT
COMPOSITION CONTAINING AN IRON COMPLEXING PROTEIN AND A PRECURSOR OF NITROGEN MONOXIDE METABOLISM AND/OR A CHEMICAL DONOR OF NITROGEN MONOXIDE AND USES THEREOF

(30) Priorité: 27.07.2000 FR 0009883
(43) Date de publication de la demande: 23.04.2003
(73) Titulaire: Isocell S.A., 92200 Neuilly-sur-Seine (FR); Dugas, Bernard, 91370 Verrières le Buisson (FR)
(72) Inventeur: DUGAS, Bernard, F-91370 Verrières le Buisson (FR)
(74) Mandataire: Goulard, Sophie
(86) Numéro de dépôt international: PCT/FR2001/002469
(87) Numéro de publication internationale: WO 2002/009740

(56) Documents cités:
- WO-A-98/33509
- US-A- 5 789 447

## Description

La présente invention est relative à une composition pharmaceutique ou complément nutritionnel contenant au moins une protéine complexant le fer, et au moins un précurseur du métabolisme du monoxyde d'azote, ainsi qu'à l'utilisation d'au moins une protéine complexant le fer et d'au moins un précurseur du métabolisme du NO pour la fabrication de compositions pharmaceutiques destinées notamment au traitement de l'asthénie et/ou de l'anémie ou pour la fabrication d'un complément nutritionnel.

Les protéines capables de complexer le fer, telles que la lactoferrine (Lf) et la transferrine (Tf) ont de nombreuses propriétés biologiques.

Il a ainsi été démontré tout d'abord (Griffiths E. *et al,* 1977, Infection & Immunity, **15** (2), 396-401) que la Lf humaine présentait une activité bactéricide.

La Lf humaine est retrouvée dans la plupart des sécrétions biologiques externes, telles que les sécrétions bronchiques, salivaires, lacrymales, nasales, gastriques, pancréatiques, duodénales, vaginales, ainsi que dans différents liquides biologiques tels que les liquides synoviale et amniotique, et enfin dans le plasma. La Lf est retrouvée dans le colostrum et le lait de la plupart des mammifères. Les concentrations de Lf dans le lait varient selon l'espèce considérée et la maturité du lait. Des études récentes, réalisées après le clonage de la Lf bovine, démontrent que la Lf bovine présente une identité de séquence protéique de 69 % (Tierce *et al.,* Eur. J. Biochem., 1991,**196**,177-184).

En fait, il apparaît que la Lf bovine présente d'autres fonctions biologiques importantes autres que son activité bactéricide et que celles-ci sont plus importantes que celles de la Lf humaine. En effet, il a été démontré que la Lf bovine présentait des propriétés immunorégulatrices et antioxydantes (Paul-Eugène *et al.,* Compte-rendu de l'Académie des Sciences françaises, 1993, **316**, 113-119). Ces observations démontrent clairement que ce type de protéines, en plus de son activité protectrice vis-à-vis des infections bactériennes, pouvait présenter un certain nombre de propriétés immuno-stimulantes et anti-oxydantes bénéfiques pour l'organisme, soit en régulant l'immunité naturelle, soit en stimulant l'hématopoïèse. Ces propriétés sont dues à la présence de récepteurs pour la Lf à la surface de plusieurs types de cellules, comme les entérocytes (Iyer et Lönnerdal, Eur. J. Clin. Nutr., 1993, 47,232-241), les cellules phagocytaires (Birgens H. S., 1991, Danish Medical Bulletin, **38** (3), 244-252) et les lymphocytes (Bennet et Davis, J. Immunol., 1981, **127**, 1211-1216 ; Birgens *et al.,* Br. J. Heamatol., 1983, **54**, 383-388).

La Lf est fortement représentée dans le lait de vache ou dans le colostrum (plusieurs mg/l). Ainsi, son utilisation sous forme semi-purifiée dans le domaine du complément nutritionnel, et purifiée dans le domaine pharmaceutique est tout à fait envisageable, notamment pour assurer le transport et la fixation du fer au niveau cellulaire en particulier dans les cas de déficit en fer, pour stimuler l'hématopoïèse dans le cas de l'anémie, pour éliminer certains agents bactériens pathogènes présents notamment dans le tube digestif, comme complément fonctionnel dans la lutte contre l'allergie et l'intolérance nutritionnelle ou bien encore en complémentation fonctionnelle de certains produits anti-oxydants tels que la superoxyde dismutase (SOD) par exemple.

Des études ont démontré l'importance de la Lf dans l'absorption intestinale du fer. En effet, le transport du fer dans les cellules épithéliales gastro-intestinales humaines (Caco-2) s'effectue préférentiellement à partir de la Lf humaine qui contient le fer sous forme complexée plutôt qu'à partir de fer sous forme libre tel que le sulfate ferreux (Cox T. M. *et al.,* 1979, Biochimica & Biophysica Acta, **558**, 129-141).

Plusieurs mécanismes de mise à disposition du fer et de la Lf ont été proposés : 1) le fer serait transporté au travers de l'entérocyte par la Lf supposée intacte (Mikogami T. *et al.,* 1994, American J. Physiol., **267**, 308-315) ; 2) le fer serait libéré au niveau de la membrane plasmique (Roiron-Lagroux et Figarella, 1990, Biochimica & Biophysica Acta, **170**, 837-842) ; 3) le fer serait libéré après adsorption de la Lf et sa dégradation lysosomiale, les produits de dégradation étant transportés par voie transcellulaire (Sanchez *et al.,* 1996, Biochimica & Biophysica Acta, **1289,** 291-297).

Des compositions pharmaceutiques contenant l'association de lactoprotéines telles que la lactoferrine et d'un sel de fer ont déjà été proposées, cependant ce type de composition bien qu'assurant le transport du fer au niveau cellulaire, n'y permet cependant pas sa fixation de façon durable.

Si le transport du fer par la Lf semble réalisable, la fixation durable du fer au niveau cellulaire reste un facteur crucial pour l'utilisation de ce type de produit dans le traitement de l'anémie notamment. Or jusqu'à ce jour, aucune composition pharmaceutique permettant à la fois le transport et la fixation du fer au niveau cellulaire n'a été proposée.

C'est afin de remédier à ce problème que les Inventeurs ont mis au point ce qui fait l'objet de l'invention.

La présente invention a donc pour objet une composition pharmaceutique ou un complément nutritionnel caractérisée par le fait qu'elle (ou qu'il) comprend à titre de principe actif :
- au moins une protéine complexant le fer, éventuellement en présence d'au moins un sel de fer, et
- au moins un précurseur du métabolisme du monoxyde d'azote (NO) et/ou au moins un donneur chimique de monoxyde d'azote ;
et éventuellement au moins un véhicule pharmaceutiquement acceptable.

Les Inventeurs ont en effet démontré que la fixation cellulaire durable du fer est fortement potentialisée en présence d'au moins un précurseur du métabolisme du NO et/ou en présence d'au moins un donneur chimique de monoxyde d'azote.

Parmi les protéines complexant le fer et pouvant être utilisées dans la composition pharmaceutique ou dans le complément nutritionnel conformes à la présente invention, on préfère utiliser de la lactoferrine (Lf) ou de la transferrine. L'utilisation de la Lf est particulièrement préférée.

La Lf peut être d'origine bovine, chevaline, caprine, ovine ou bien encore être obtenue par voie synthétique ou recombinante. Parmi les Lf recombinantes, on peut notamment citer celles qui sont décrites dans les Demandes Internationales WO 97/45136 et WO 98/50543.

D'une manière générale, les protéines complexant le fer peuvent être utilisées sous forme native ou sous forme modifiée afin d'augmenter leur taux de saturation en fer.

A titre d'exemple, et lorsque la Lf est utilisée, celle-ci peut ainsi subir un traitement à pH acide en présence d'un sel de fer de façon à augmenter son taux de saturation en fer à des valeurs supérieures à 80 % environ, comparativement à la Lf à l'état naturel (Lf native) qui présente un taux de saturation en fer de l'ordre de 20 % environ.

Selon une forme de réalisation avantageuse de l'invention, on préfère utiliser une lactoferrine présentant un taux de saturation en fer supérieur à 50 % et encore plus préférentiellement supérieur à 80 %.

La ou les protéines complexant le fer sont de préférence utilisées à des doses unitaires comprises entre 0,01 mg et 5 mg environ, et encore plus préférentiellement entre 0,1 mg et 1 mg environ.

Parmi les sels de fer pouvant éventuellement être utilisés dans la composition pharmaceutique ou dans le complément nutritionnel conformes à l'invention, on peut notamment citer les sels les sels ferreux (sulfate, chlorure), les sels ferriques (chlorure, sulfate, phosphate), le fumarate de fer, l'oxalate de fer, l'ascorbate de fer, le gluconate de fer, le succinate de fer, l'acétate de fer, et le ferédétate.

Lorsqu'ils sont utilisés, le ou les sels de fer peuvent être présents au sein de la composition pharmaceutique ou au sein du complément nutritionnel conformes à l'invention soit sous forme non complexée, c'est-à-dire que le sel de fer n'est pas complexé à la protéine complexant le fer, soit sous forme partiellement complexée ou totalement complexée à la protéine complexant le fer.

Lorsqu'ils sont présents au sein de la composition phannaceutique ou au sein du complément nutritionnel conformes à l'invention, le ou les sels de fer sont de préférence utilisés à des doses unitaires comprises entre 0,01 et 1 mg environ et encore plus préférentiellement entre 0,05 et 0,5 mg environ.

Parmi les précurseurs du métabolisme du NO, on peut citer la L-arginine et ses sels tels que le chlorhydrate de L-arginine, le glutamate de L-arginine, et l'aspartate de L-arginine ; les protéines et les peptides riches en L-arginine, c'est-à-dire constitués d'au moins 10 % en poids de L-arginine, et parmi lesquels on peut citer notamment les protéines et les peptides issus d'extraits de lupin.

Parmi les donneurs chimiques de monoxyde d'azote, on peut citer la S-nitroso-N-acétyl-pénicillamine (SNAP).

Le ou les précurseurs du métabolisme du NO et/ou le ou les donneurs chimiques de NO sont de préférence utilisés à des doses unitaires comprises entre 0,001 mg et 1 mg environ et encore plus particulièrement entre 0,01 mg et 0,1 mg environ.

Lorsque de la L-arginine et/ou l'un de ses sels sont présents dans la composition pharmaceutique ou dans le complément nutritionnel conformes à l'invention, ils sont alors utilisés à des doses unitaires comprises de préférence entre 0,1 mg et 5 mg environ et encore plus particulièrement entre 0,5 et 2 mg environ.

Selon l'invention le rapport pondéral protéine(s) complexant le fer / précurseur(s) du métabolisme du NO et/ou donneur(s) chimique(s) de NO est de préférence compris entre 1/1 et 10/1.

Selon une forme de réalisation particulièrement préférée de l'invention, le rapport protéine(s) complexant le fer / précurseur(s) du métabolisme du NO et/ou donneur(s) chimique(s) de NO est de 1/1 (poids pour poids).

Selon l'invention, on préfère une composition pharmaceutique ou un complément nutritionnel renfermant l'association de :
- de 0,01 mg à 5 mg de lactoferrine,
- de 0,01 à 1 mg d'un sel de fer, et
- de 0,1 mg à 5 mg de L-arginine.

Selon l'invention, on préfère encore plus particulièrement une composition pharmaceutique ou un complément nutritionnel renfermant l'association de :
- de 0,01 mg à 1 mg de lactoferrine,
- 0,1 mg d'un sel de fer, et
- 1 mg de L-arginine.

La composition pharmaceutique conforme à l'invention présente les propriétés de pouvoir être notamment utilisées pour stimuler l'hématopoïèse dans les cas d'anémie, et pour le traitement de l'asthénie. De plus, grâce aux propriétés biologiques intrinsèques des lactoferrines, la composition pharmaceutique conforme à l'invention, lorsqu'elle renferme de la lactoferrine à titre de protéine complexant le fer, peut également être utilisée pour l'élimination d'agents bactériens pathogènes (dans le tube digestif en particulier), comme complément fonctionnel dans la lutte contre l'allergie et l'intolérance nutritionnelle ou bien encore comme complément fonctionnel en association avec certains produits anti-oxydants tels que la SOD en particulier.

A ce titre, une composition pharmaceutique ou un complément nutritionnel conforme à l'invention, renfermant au moins une lactoferrine à titre de protéine complexant le fer et en outre de la SOD, est particulièrement préférée.

Le complément nutritionnel conforme à l'invention présente la propriété de pouvoir être utilisé pour prévenir les déficits en fer ou les risques de déficit en fer, notamment chez la femme enceinte.

La composition pharmaceutique conforme à l'invention peut également renfermer un ou plusieurs principes actifs additionnels tels que ceux habituellement utilisés dans les compositions pharmaceutiques destinées au traitement de l'asthénie et/ou de l'anémie, et parmi lesquels on peut notamment citer les vitamines telles que la vitamine B₁₂, l'acide folique (ou vitamine B_{c}), et l'acide ascorbique (ou vitamine C) ; le co-enzyme Q10.

Le véhicule pharmaceutiquement acceptable selon l'invention est de préférence constitué par un ou plusieurs excipients classiquement utilisés pour la préparation de compositions pharmaceutiques, tels que des anti-agglomérants, des anti-oxydants, des colorants, des agents modifiants du goût, des agents de lissage, de montage, d'isolation et de manière générale, tout excipient classiquement utilisé dans l'industrie pharmaceutique.

Bien entendu, l'homme de l'art veillera à cette occasion à ce que le ou les excipients éventuellement utilisés soient compatibles avec les propriétés intrinsèques attachées à la présente invention.

La composition pharmaceutique conforme à l'invention est de préférence administrée par voie orale à raison de une à deux prises par jour pendant 8 jours.

Elle peut se présenter sous diverses formes, telles que sous forme de comprimés, de gélules, de suspensions buvables, de tablettes ou sous toute autre forme appropriée au mode d'administration par voie orale,

Le complément nutritionnel conforme à l'invention est de préférence absorbé par voie orale et peut se présenter sous diverses formes, telles que de comprimés, de gélules, de suspensions buvables, de tablettes ou sous toute autre forme appropriée à la présentation habituelle d'un complément nutritionnel.

L'invention a également pour objet l'utilisation d'au moins une protéine complexant le fer et d'au moins un précurseur du métabolisme du NO et/ou d'au moins un donneur chimique de monoxyde d'azote, et éventuellement d'au moins un sel de fer, pour la préparation d'une composition pharmaceutique, celle-ci étant notamment destinée au traitement de l'asthénie et/ou de l'anémie.

L'invention a également pour objet l'utilisation d'au moins une protéine complexant le fer et d'au moins un précurseur du métabolisme du NO et/ou d'au moins un donneur chimique de monoxyde d'azote, et éventuellement d'au moins un sel de fer, pour la préparation d'un complément nutritionnel destiné à des sujets présentant un déficit en fer ou un risque de déficit en fer, notamment la femme enceinte.

Outre les dispositions qui précèdent, l'invention comprend encore d'autres dispositions qui ressortiront de la description qui va suivre, qui se réfère à des exemples de démonstration de la potentialisation de la fixation du fer dans des cellules épithéliales gastro-intestinales humaines, ainsi qu'aux figures annexées, dans lesquelles :
- la figure 1 montre les effets de l'addition d'un précurseur du métabolisme du NO sur la fixation du fer au niveau cellulaire ;
- la figure 2 montre les effets de l'addition d'un inhibiteur de la voie de la L-arginine sur le taux de fixation du fer au niveau cellulaire ; et

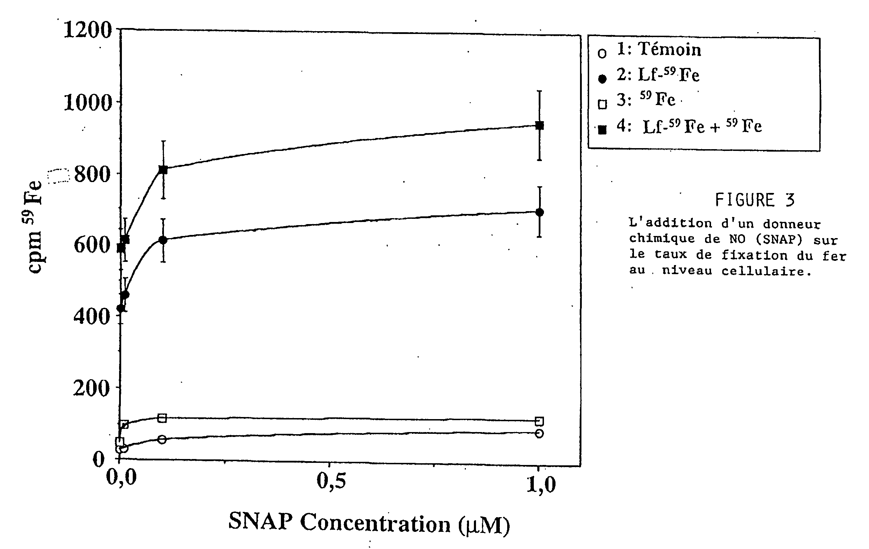

- la figure 3 montre les effets de l'addition d'un donneur chimique de NO (SNAP) sur le taux de fixation du fer au niveau cellulaire.

Il doit être bien entendu toutefois que ces exemples sont donnés uniquement à titre d'illustration de l'objet de l'invention, dont ils ne constituent en aucune manière une limitation.

### EXEMPLE 1 : DÉMONSTRATION DE L'EFFET DE POTENTIALISATION DE LA FIXATION DU FER AU NIVEAU CELLULAIRE PAR UN PRÉCURSEUR, DU MÉTABOLISME DU NO

Le but de cet exemple est de démontrer que la fixation du fer par la Lf au niveau cellulaire est fortement potentialisée en présence d'un précurseur du métabolisme du NO tel que la L-arginine.

A l'état naturel, la Lf bovine (Lf bovine native) présente une saturation en fer de l'ordre de 20 % environ. De la Lf bovine native a été traitée à pH 1 de manière à substituer le fer non marqué qu'elle renfermait à l'état naturel par du fer marqué ⁵⁹Fe. La saturation de la Lf native par du fer marqué a abouti à une lactoferrine marquée (Lf-⁵⁹Fe) présentant un taux de saturation en fer marqué de l'ordre de 85 % à 100 %, révélé par absorption spectrophotométrique à 450 nm pour une activité spécifique de 0,03 µCi/µg.

La Lf-⁵⁹Fe a ensuite été purifiée pour éliminer le fer marqué non fixé, par filtration sur gel, sur une colonne de SEPHADEX ® G-75.

Des cellules épithéliales intestinales de la lignée Caco-2 dérivée d'un cancer du colon sont incubées à raisons de 2.10⁶ cellules par ml dans un milieu RPMI 1640 tamponné à pH 7,4 en présence de 25 mmolaire de Hepes, contenant 1 % (v/v) de sérum de veau foetal, 2 mmol/l de L-glutamine et 1 % (v/v) d'une solution de streptomycine-pénicilline, vendu par la société Gibco.

La fixation du fer au niveau cellulaire est ensuite initiée en rajoutant de la Lf-⁵⁹Fe et/ou du fer marqué et/ou de la L-arginine.

Un des tubes de culture de cellules ne reçoit aucun de ces trois constituants, de façon à servir de témoin.

La répartition des constituants, ainsi que les concentrations auxquelles ils sont utilisés figurent dans le Tableau I ci-après :

**TABLEAU I**

| **Tubes de cultures de cellules** | **Lf-**^{**59**}**Fe** | **Fer radiomarqué (**^{**59**}**Fe)** | **L-arginine** |
|---|---|---|---|
| **1 (témoin)** | **-** | **-** | **-** |
| **2** | 1 µg | - | - |
| **3** | - | - | 10 mM |
| **4** | 1 µg | - | 10 mM |
| **5** | - | 100 µg | - |
| **6** | 1 µg | 100 µg | - |
| **7** | - | 100 µg | 10 mM |
| **8** | 1 µg | 100 µg | 10 mM |

Les cellules sont ensuite incubées pendant une période de 16 heures dans des tubes Eppendorf maintenus en agitation pendant les 2 dernières heures de la période de culture. Durant toute la période d'incubation, le taux de fer cellulaire a été mesuré sur culot de centrifugation des cellules une première fois au bout de 30 minutes (t=0,5) puis au bout d'une heure (t=1), et ensuite toutes les heures (t=2 à t=16), à l'aide d'un appareil de mesure de la radioactivité.

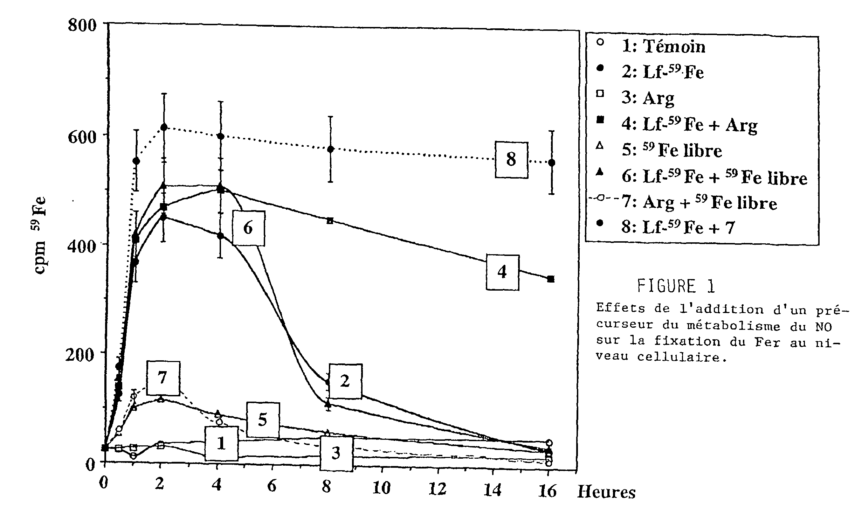

Ainsi que cela peut être observé sur la figure 1, la fixation du fer par les cellules intestinales est observée à partir de t=0,5, avec un maximum entre t=1 et t=2, pour ensuite diminuer jusqu'à t=16. Cet effet démontre que la Lf-⁵⁹Fe est capable de transporter le fer marqué dans la cellule cible, cependant celui-ci n'y est fixé que de manière transitoire puisque le taux de fer radioactif cellulaire diminue par la suite, vraisemblablement par un phénomène de fuite cellulaire. Dans le cas où ces cultures cellulaires sont complémentées par de la L-arginine et éventuellement par du fer radiomarqué (cultures de cellules 4 et 8), on note une augmentation du taux de fixation du fer au niveau cellulaire ainsi qu'une prolongation de la durée de fixation du fer jusqu'après 16 heures de culture.

L'addition d'un précurseur du métabolisme du NO à une composition pharmaceutique contenant une protéine complexant le fer et éventuellement un sel de fer permet donc non seulement d'augmenter la quantité de fer fixé au niveau cellulaire mais également de prolonger la durée de fixation du fer au niveau cellulaire, cet effet étant d'autant plus marqué lorsque ladite composition contient à la fois l'association des trois constituants.

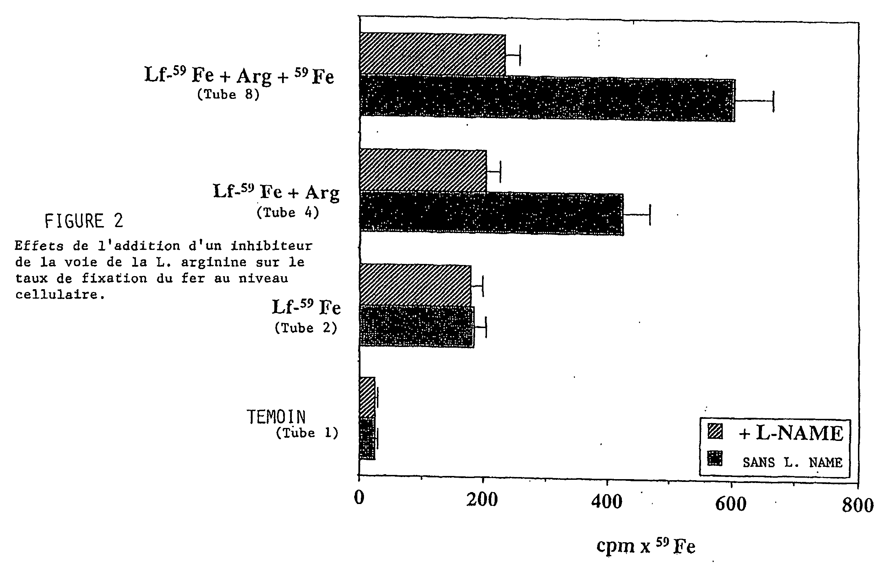

D'autre part, et ainsi que cela apparaît sur la figure 2, si on répète l'expérience réalisée avec les tubes de cultures de cellules 1, 2, 4 et 8 qu'un inhibiteur compétitif de la voie de la L-arginine à savoir le méthyl ester de la N^{ω}-nitro-L-arginine (le L-NAME) est ajouté à raison de 1mM dans le milieu de culture cellulaire de ces tubes, il apparaît que cette addition entraîne une diminution très importante du taux de fixation du fer au niveau cellulaire, celui-ci étant pratiquement ramené au taux qui est observé lorsque l'incubation des cellules est réalisée sans addition de L-arginine (tube 2). Cette expérience montre qu'en bloquant le métabolisme de la L-arginine par l'utilisation de L-NAME, on bloque la génération de NO par les cellules mises en culture, ce qui indique que le NO endogène est responsable de l'augmentation du taux de fixation du fer au niveau cellulaire.

### EXEMPLE 2 : DÉMONSTRATION DE L'EFFET DE POTENTIALISATION DE LA FIXATION DU FER AU NIVEAU CELLULAIRE PAR UN DONNEUR CHIMIQUE DE NO

Le but de cet exemple est de démontrer que la fixation du fer par la Lf au niveau cellulaire est fortement potentialisée en présence d'un donneur chimique de NO tel que la SNAP.

Des tubes de cultures de cellules épithéliales intestinales ont été préparées et cultivées dans les mêmes conditions que celles décrites ci-dessus à l'exemple 1.

La fixation du fer au niveau cellulaire est ensuite initiée en rajoutant de la Lf-⁵⁹Fe et/ou du fer marqué.

Un des tubes de culture de cellules ne reçoit aucun des constituants, de façon à servir de témoin.

La répartition des constituants, ainsi que les concentrations auxquelles ils sont utilisés figurent dans le Tableau II ci-après :

**TABLEAU II**

| **Tubes de cultures de cellules** | **Lf-**^{**59Fe**} | **Fer radiomarqué (**^{**59**}**Fe)** |
|---|---|---|
| **1 (témoin)** | - | - |
| **2** | 1 µg | - |
| **3** | - | 100 µg |
| **4** | 1 µg | 100 µg |

Les tubes de cultures reçoivent ensuite de la SNAP en des quantités variant entre 0 et 1 µM et la fixation cellulaire de fer radiomarqué est ensuite mesurée à l'aide d'un appareil de mesure de la radioactivité.

Ainsi que cela peut être observé sur la figure 2, l'addition de SNAP, à une composition renfermant de la Lf (tubes de cultures de cellules 2) et éventuellement du fer radiomarqué (tubes de cultures de cellules 4) permet d'augmenter le taux de fixation du fer au niveau des cellules épithéliales.

Par conséquent, l'addition d'un donneur chimique de NO à une composition pharmaceutique contenant une protéine complexant le fer et éventuellement un sel de fer permet d'augmenter le taux de fixation du fer au niveau cellulaire ; cet effet étant plus marqué lorsque ladite composition contient à la fois l'association des trois constituants.

### EXEMPLE 3 : ÉTUDE DU TAUX DE FIXATION DU FER AU NIVEAU CELLULAIRE EN FONCTION DES CONCENTRATIONS

Selon les conditions décrites ci-dessus à l'exemple 1, 12 tubes de cultures de cellules épithéliales ont été préparés.

La fixation du fer au niveau cellulaire est ensuite initiée en rajoutant de la Lf-⁵⁹Fe et/ou du fer marqué et/ou de la L-arginine.

Un des tubes de culture de cellules ne reçoit aucun de ces trois constituants, de façon à servir de témoin.

La répartition des constituants, ainsi que les concentrations auxquelles ils sont utilisés figurent dans le tableau III ci-après :

**TABLEAU III**

| **Tubes de cultures de cellules** | **Lf-**^{**59**}**Fe en mg/ml** | **Fer radiomarqué (**^{**59**}**Fe) en mg/ml** | **L-arginine en mg/ml** |
|---|---|---|---|
| **Témoin** | - | - | - |
| **1** | 1 | - | - |
| **2** | 0,1 | - | - |
| **3** | 0,01 | - | - |
| **4** | - | 0,1 | - |
| **5** | 1 | 0,1 | - |
| **6** | 0,1 | 0,1 | - |
| **7** | 0,01 | 0,1 | - |
| **8** | - | - | 1 |
| **9** | 1 | 0,1 | 1 |
| **10** | 0,1 | 0,1 | 1 |
| **11** | 0,01 | 0,1 | 1 |

Le taux de fixation cellulaire du fer a été mesuré au bout de 72 heures d'incubation au moyen d'un appareil de mesure de la radioactivité.

Les résultats obtenus pour chacun des 12 tubes figurent dans le tableau IV ci-après :

**TABLEAU IV**

| **Tubes de cultures de cellules** | **Taux de fixation du fer dans les cellules (cpm** ^{**59**}**Fe)** |
|---|---|
| **Témoin** | 1 |
| **1** | 1,2 ± 0,1 |
| **2** | 1,0 ± 0,2 |
| **3** | 1,1 ± 0,1 |
| **4** | 1,8 ± 0,1 |
| **5** | 2,1 ± 0,2 |
| **6** | 1,9 ± 0,1 |
| **7** | 1,7 ± 0,1 |
| **8** | 1 |
| **9** | **5,4 ± 0,3** |
| **10** | **4,3 ± 0,2** |
| **11** | **3,1 ± 0,1** |

Ces résultats montrent qu'aux trois concentrations en Lf testées, on observe une très importante augmentation du taux de fixation du fer au niveau cellulaire lorsque le milieu de culture cellulaire renferme conjointement une protéine complexant le fer, un sel de fer et un précurseur du métabolisme du NO, prouvant ainsi le rôle essentiel que joue la L-arginine sur la potentialisation de la fixation du fer par les cellules.

## Revendications

1. Composition pharmaceutique ou complément nutritionnel **caractérisé**(e) par le fait qu'elle (qu'il) comprend à titre de principe actif :
- au moins une protéine complexant le fer, éventuellement en présence d'au moins un sel de fer, et
- au moins un précurseur du métabolisme du monoxyde d'azote et/ou au moins un donneur chimique de monoxyde d'azote,
et éventuellement au moins un véhicule pharmaceutiquement acceptable.

2. Composition pharmaceutique ou complément nutritionnel selon la revendication 1, **caractérisé**(e) par le fait que la ou les protéines complexant le fer sont choisies parmi lactoferrine et la transferrine.

3. Composition pharmaceutique ou complément nutritionnel selon la revendication 2, **caractérisé**(e) par le fait qu'elle (qu'il) renferme une lactoferrine d'origine bovine, chevaline, caprine, ovine ou une lactoferrine obtenue par voie synthétique ou recombinante.

4. Composition pharmaceutique ou complément nutritionnel selon la revendication 3, **caractérisé**(e) par le fait qu'elle (qu'il) renferme une lactoferrine présentant un taux de saturation en fer supérieur à 50 %.

5. Composition pharmaceutique ou complément nutritionnel selon l'une quelconque des revendications précédentes, **caractérisé**(e) par le fait que la ou les protéines complexant le fer sont utilisées à des doses unitaires comprises entre 0,01 mg et 5 mg.

6. Composition pharmaceutique ou complément nutritionnel selon la revendication 5, **caractérisé**(e) par le fait que la ou les protéines complexant le fer sont utilisées à des doses unitaires comprises entre 0,1 mg et 1 mg.

7. Composition pharmaceutique ou complément nutritionnel selon l'une quelconque des revendications précédentes, **caractérisé**(e) par le fait que les sels de fer sont choisis parmi les sels ferreux, les sels ferriques, le fumarate de fer, l'oxalate de fer, l'ascorbate de fer, le gluconate de fer, le succinate de fer, l'acétate de fer et le ferédétate.

8. Composition pharmaceutique ou complément nutritionnel selon l'une quelconque des revendications précédentes, **caractérisé**(e) par le fait que le ou les sels de fer sont présents au sein de ladite composition ou dudit complément nutritionnel soit sous forme complexée, soit sous forme partiellement complexée ou totalement complexée, à ladite protéine complexant le fer.

9. Composition pharmaceutique ou complément nutritionnel selon l'une quelconque des revendications précédentes, **caractérisé**(e) par le fait que le ou les sels de fer sont utilisés à des doses unitaires comprises entre 0,01 et 1 mg.

10. Composition pharmaceutique ou complément nutritionnel selon la revendication 9, **caractérisé**(e) par le fait que le ou les sels de fer sont utilisés à des doses unitaires comprises entre 0,05 et 0,5 mg.

11. Composition pharmaceutique ou complément nutritionnel selon l'une quelconque des revendications précédentes, **caractérisé**(e) par le fait que le ou les précurseurs du métabolisme du monoxyde d'azote sont choisis parmi la L-arginine et ses sels ; les protéines et les peptides constitués d'au moins 10 % en poids de L-arginine.

12. Composition pharmaceutique ou complément nutritionnel selon la revendication 11, **caractérisé**(e) par le fait que les sels de L-arginine sont choisis parmi le chlorhydrate de L-arginine, le glutamate de L-arginine, et l'aspartate de L-arginine.

13. Composition pharmaceutique ou complément nutritionnel selon la revendication 11, **caractérisé**(e) par le fait que lesdites protéines et lesdits peptides sont issus d'extraits de lupin.

14. Composition pharmaceutique ou complément nutritionnel selon l'une quelconque des revendications précédentes, **caractérisé**(e) par le fait que le donneur chimique de monoxyde d'azote est de la S-nitroso-N-acétyl-pénicillamine.

15. Composition pharmaceutique ou complément nutritionnel selon l'une quelconque des revendications précédentes, **caractérisé**(e) par le fait que le ou les précurseurs du métabolisme du monoxyde d'azote et/ou le ou les donneurs chimiques de monoxyde d'azote sont utilisés à des doses unitaires comprises entre 0,001 mg et 1 mg.

16. Composition pharmaceutique ou complément nutritionnel selon la revendication 15, **caractérisé**(e) par le fait que le ou les précurseurs du métabolisme du monoxyde d'azote et/ou le ou les donneurs chimiques de monoxyde d'azote sont utilisés à des doses unitaires comprises entre 0,01 mg et 0,1 mg.

17. Composition pharmaceutique ou complément nutritionnel selon l'une quelconque des revendications précédentes, **caractérisé**(e) par le fait que le rapport pondéral protéine(s) complexant le fer / précurseur(s) du métabolisme du monoxyde d'azote et/ou donneur(s) chimique(s) de monoxyde d'azote est de préférence compris entre 1/1 et 10/1.

18. Composition pharmaceutique ou complément nutritionnel selon la revendication 17, **caractérisé**(e) par le fait que le rapport pondéral protéine(s) complexant le fer / précurseur(s) du métabolisme du monoxyde d'azote et/ou donneur(s) chimique(s) de monoxyde d'azote est de 1/1.

19. Composition pharmaceutique ou complément nutritionnel selon l'une quelconque des revendications précédentes, **caractérisé**(e) par le fait qu'elle (qu'il) renferme l'association de :
- de 0,01 mg à 5 mg de lactoferrine,
- de 0,01 à 1 mg d'un sel de fer, et
- de 0,1 mg à 5 mg de L-arginine.

20. Composition pharmaceutique ou complément nutritionnel selon la revendication 19, **caractérisé**(e) par le fait qu'elle (qu'il) renferme l'association de:
- de 0,01 mg à 1 mg de lactoferrine,
- 0,1 mg d'un sel de fer, et
- 1 mg de L-arginine.

21. Composition pharmaceutique ou complément nutritionnel selon l'une quelconque des revendications précédentes, **caractérisé**(e) par le fait qu'elle (qu'il) renferme en outre de la superoxyde dismutase.

22. Utilisation d'au moins une protéine complexant le fer et d'au moins un précurseur du métabolisme du monoxyde d'azote et/ou d'au moins un donneur chimique de monoxyde d'azote, et éventuellement d'au moins un sel de fer, pour la préparation d'une composition pharmaceutique notamment destinée au traitement de l'asthénie et/ou de l'anémie.

23. Utilisation d'au moins une protéine complexant le fer et d'au moins un précurseur du métabolisme du monoxyde d'azote et/ou d'au moins un donneur chimique de monoxyde d'azote, et éventuellement d'au moins un sel de fer, pour la préparation d'un complément nutritionnel.

## Patentansprüche

1. Pharmazeutische Zusammensetzung oder Nahrungsergänzungsmittel, **dadurch gekennzeichnet, daß** sie / es als Wirkstoff
- mindestens ein eisenkomplexierendes Protein, eventuell bei Vorhandensein von mindestens einem Eisensalz und
- mindestens einer metabolischen Vorstufe des Stickstoffmonoxids und/oder mindestens einen chemischen Stickstoffmonoxid-Donor und
- eventuell mindestens einen pharmazeutisch akzeptablen Träger
enthält.

2. Pharmazeutische Zusammensetzung oder Nahrungsergänzungsmittel nach Anspruch 1, **dadurch gekennzeichnet, daß** das oder die eisenkomplexierende(n) Protein(e) aus Laktoferrin und Transferrin ausgewählt sind.

3. Pharmazeutische Zusammensetzung oder Nahrungsergänzungsmittel nach Anspruch 2, **dadurch gekennzeichnet, daß** sie / es ein vom Rind, vom Pferd, von der Ziege, vom Schaf gewonnenes Laktoferrin oder ein synthetisch oder durch Rekombination erhaltenes Laktoferrin enthält.

4. Pharmazeutische Zusammensetzung oder Nahrungsergänzungsmittel nach Anspruch 3, **dadurch gekennzeichnet, daß** sie / es ein Laktoferrin mit einer Eisensättigung über 50 % enthält.

5. Pharmazeutische Zusammensetzung oder Nahrungsergänzungsmittel nach einem der vorhergehenden Ansprüche, **dadurch gekennzeichnet, daß** das oder die eisenkomplexierende(n) Protein(e) in Einheitsdosen zwischen 0,01 mg und 5 mg eingesetzt werden.

6. Pharmazeutische Zusammensetzung oder Nahrungsergänzungsmittel nach Anspruch 5, **dadurch gekennzeichnet, daß** das oder die eisenkomplexierende(n) Protein(e) in Einheitsdosen zwischen 0,1 mg und 1 mg eingesetzt werden.

7. Pharmazeutische Zusammensetzung oder Nahrungsergänzungsmittel nach einem der vorhergehenden Ansprüche, **dadurch gekennzeichnet, daß** die Eisensalze unter den Eisen-II-Salzen, Eisen-III-Salzen, Eisenfumarat, Eisenoxalat, Eisenascorbat, Eisengluconat, Eisensuccinat, Eisenacetat und Eisen(III)-EDTA ausgewählt werden.

8. Pharmazeutische Zusammensetzung oder Nahrungsergänzungsmittel nach einem der vorhergehenden Ansprüche, **dadurch gekennzeichnet, daß** das oder die Eisensalz(e) in der genannten Zusammensetzung oder dem genannten Ergänzungsmittel entweder nicht komplexiert, teilweise komplexiert oder vollständig komplexiert mit dem genannten eisenkomplexierenden Protein vorhanden sind.

9. Pharmazeutische Zusammensetzung oder Nahrungsergänzungsmittel nach einem der vorhergehenden Ansprüche, **dadurch gekennzeichnet, daß** das oder die Eisensalz(e) in Einheitsdosen zwischen 0,01 mg und 1 mg eingesetzt werden.

10. Pharmazeutische Zusammensetzung oder Nahrungsergänzungsmittel nach Anspruch 9, **dadurch gekennzeichnet, daß** das oder die Eisensalz(e) in Einheitsdosen zwischen 0,05 mg und 0,5 mg eingesetzt werden.

11. Pharmazeutische Zusammensetzung oder Nahrungsergänzungsmittel nach einem der vorhergehenden Ansprüche, **dadurch gekennzeichnet, daß** der oder die metabolischen Vorstufen des Stickstoffmonoxids aus L-Arginin und dessen Salzen; den aus mindestens 10 Gew.% L-Arginin bestehenden Proteinen und Peptiden ausgewählt sind.

12. Pharmazeutische Zusammensetzung oder Nahrungsergänzungsmittel nach Anspruch 11, **dadurch gekennzeichnet, daß** die L-Arginin-Salze aus L-Arginin-Chlorhydrat, L-Arginin-Glutamat und L-Arginin-Aspartat ausgewählt sind.

13. Pharmazeutische Zusammensetzung oder Nahrungsergänzungsmittel nach Anspruch 11, **dadurch gekennzeichnet, daß** die genannten Proteine und die genannten Peptide aus Lupinenextrakten gewonnen werden.

14. Pharmazeutische Zusammensetzung oder Nahrungsergänzungsmittel nach einem der vorhergehenden Ansprüche, **dadurch gekennzeichnet, daß** der chemische Stickstoffmonoxid-Donor S-Nitroso-N-acetyl-penicillamin ist.

15. Pharmazeutische Zusammensetzung oder Nahrungsergänzungsmittel nach einem der vorhergehenden Ansprüche, **dadurch gekennzeichnet, daß** der oder die Vorläufer im Stoffwechsel des Stickstoffmonoxids und/oder der oder die chemische(n) Stickstoffmonoxid-Donor(en) in Einheitsdosen zwischen 0,001 mg und 1 mg eingesetzt werden.

16. Pharmazeutische Zusammensetzung oder Nahrungsergänzungsmittel nach Anspruch 15, **dadurch gekennzeichnet, daß** der oder die Vorläufer im Stoffwechsel des Stickstoffmonoxids und/oder der oder die chemische(n) Stickstoffmonoxid-Donor(en) in Einheitsdosen zwischen 0,01 mg und 0,1 mg eingesetzt werden.

17. Pharmazeutische Zusammensetzung oder Nahrungsergänzungsmittel nach einem der vorhergehenden Ansprüche, **dadurch gekennzeichnet, daß** das Gewichtsverhältnis von eisenkomplexierendem Protein bzw. eisenkomplexierenden Proteinen zu metabolischen Vorstufen des Stickstoffmonoxids und/oder chemischem Stickstoffmonoxid-Donor / chemischen Stickstoffmonoxid-Donoren vorzugsweise zwischen 1/1 und 10/1 liegt.

18. Pharmazeutische Zusammensetzung oder Nahrungsergänzungsmittel nach Anspruch 17, **dadurch gekennzeichnet, daß** das Gewichtsverhältnis von eisenkomplexierendem Protein / eisenkomplexierenden Proteinen zu metabolischen Vorstufen und/oder chemischen Stickstoffmonoxid-Donor / chemischen Stickstoffmonoxid-Donoren 1/1 beträgt.

19. Pharmazeutische Zusammensetzung oder Nahrungsergänzungsmittel nach einem der vorhergehenden Ansprüche, **dadurch gekennzeichnet, daß** sie / es die Kombination von:
- 0,01 mg bis 5 mg Laktoferrin,
- 0,01 bis 1 mg eines Eisensalzes und
- 0,1 mg bis 5 mg L-Arginin
enthält.

20. Pharmazeutische Zusammensetzung oder Nahrungsergänzungsmittel nach Anspruch 19, **dadurch gekennzeichnet, daß** sie / es die Kombination von:
- 0,01 mg bis 1 mg Laktoferrin,
- 0,1 mg eines Eisensalzes und
- 1 mg L-Arginin
enthält.

21. Pharmazeutische Zusammensetzung oder Nahrungsergänzungsmittel nach einem der vorhergehenden Ansprüche, **dadurch gekennzeichnet, daß** sie / es ferner Superoxid-Dismutase enthält.

22. Verwendung mindestens eines eisenkomplexierenden Proteins und mindestens einer metabolischen Vorstufe des Stickstoffmonoxids und/oder mindestens eines chemischen Stickstoffmonoxid-Donors und eventuell mindestens eines Eisensalzes für die Herstellung einer pharmazeutischen Zusammensetzung insbesondere für die Behandlung der Asthenie und/oder der Anämie.

23. Verwendung mindestens eines eisenkomplexierenden Proteins und mindestens einer metabolischen Vorstufe des Stickstoffmonoxids und/oder mindestens eines chemischen Stickstoffmonoxid-Donors und eventuell mindestens eines Eisensalzes für die Herstellung eines Nahrungsergänzungsmittels.

## Claims

1. Pharmaceutical composition or nutritional supplement, **characterised in that** it contains as active ingredient:
- at least one iron complexing protein, optionally in the presence of at least one iron salt, and
- at least one precursor of the metabolism of nitrogen monoxide and/or at least one chemical donor of nitrogen monoxide,
and optionally at least one pharmaceutically acceptable carrier.

2. Pharmaceutical composition or nutritional supplement according to claim 1, **characterised in that** the iron complexing protein or proteins is or are selected from lactoferrin and transferrin.

3. Pharmaceutical composition or nutritional supplement according to claim 2, **characterised in that** it contains a lactoferrin of bovine, equine, caprine or ovine origin or a lactoferrin obtained synthetically or by recombinant methods.

4. Pharmaceutical composition or nutritional supplement according to claim 3, **characterised in that** it contains a lactoferrin having an iron saturation level greater than 50%.

5. Pharmaceutical composition or nutritional supplement according to any one of the preceding claims, **characterised in that** the iron complexing protein or proteins is or are used in single doses of between 0.01 mg and 5 mg.

6. Pharmaceutical composition or nutritional supplement according to claim 5, **characterised in that** the iron complexing protein or proteins is or are used in single doses of between 0.1 mg and 1 mg.

7. Pharmaceutical composition or nutritional supplement according to any one of the preceding claims, **characterised in that** the iron salts are selected from among the ferrous salts, the ferric salts, iron fumarate, iron oxalate, iron ascorbate, iron gluconate, iron succinate, iron acetate and iron edetate.

8. Pharmaceutical composition or nutritional supplement according to any one of the preceding claims, **characterised in that** the iron salt or salts are present within said composition or said nutritional supplement either in complexed form or in partially or totally complexed form with said iron complexing protein.

9. Pharmaceutical composition or nutritional supplement according to any one of the preceding claims, **characterised in that** the iron salt or salts is or are used in single doses of between 0.01 and 1 mg.

10. Pharmaceutical composition or nutritional supplement according to claim 9, **characterised in that** the iron salt or salts is or are used in single doses of between 0.05 and 0.5 mg.

11. Pharmaceutical composition or nutritional supplement according to any one of the preceding claims, **characterised in that** the precursor or precursors of the metabolism of nitrogen monoxide are selected from L-arginine and the salts thereof; the proteins and peptides consisting of at least 10 wt.% of L-arginine.

12. Pharmaceutical composition or nutritional supplement according to claim 11, **characterised in that** the L-arginine salts are selected from among L-arginine hydrochloride, L-arginine glutamate and L-arginine aspartate.

13. Pharmaceutical composition or nutritional supplement according to claim 11, **characterised in that** said proteins and said peptides are obtained from lupin extracts.

14. Pharmaceutical composition or nutritional supplement according to any one of the preceding claims, **characterised in that** the chemical donor of nitrogen monoxide is S-nitroso-N-acetyl-penicillamine.

15. Pharmaceutical composition or nutritional supplement according to any one of the preceding claims, **characterised in that** the precursor or precursors of the metabolism of nitrogen monoxide and/or the chemical donor or donors of nitrogen monoxide is or are used in single doses of between 0.001 mg and 1 mg.

16. Pharmaceutical composition or nutritional supplement according to claim 15, **characterised in that** the precursor or precursors of the metabolism of nitrogen monoxide and/or the chemical donor or donors of nitrogen monoxide is or are used in single doses of between 0.01 mg and 0.1 mg.

17. Pharmaceutical composition or nutritional supplement according to any one of the preceding claims, **characterised in that** the weight ratio of iron complexing protein(s) to precursor(s) of the metabolism of nitrogen monoxide and/or chemical donor(s) of nitrogen monoxide is preferably between 1:1 and 10:1.

18. Pharmaceutical composition or nutritional supplement according to claim 17, **characterised in that** the weight ratio of iron complexing protein(s) to precursor(s) of the metabolism of nitrogen monoxide and/or chemical donor(s) of nitrogen monoxide is 1:1.

19. Pharmaceutical composition or nutritional supplement according to any one of the preceding claims, **characterised in that** it contains a combination of:
- 0.01 mg to 5 mg of lactoferrin,
- 0.01 to 1 mg of an iron salt, and
- 0.1 mg to 5 mg of L-arginine.

20. Pharmaceutical composition or nutritional supplement according to claim 19, **characterised in that** it contains a combination of:
- 0.01 mg to 1 mg of lactoferrin,
- 0.1 mg of an iron salt, and
- 1 mg of L-arginine.

21. Pharmaceutical composition or nutritional supplement according to any one of the preceding claims, **characterised in that** it further contains superoxide dismutase.

22. Use of at least one iron complexing protein and at least one precursor of the metabolism of nitrogen monoxide and/or at least one chemical donor of nitrogen monoxide, and optionally at least one iron salt, for the preparation of a pharmaceutical composition intended in particular for the treatment of asthenia and/or anaemia.

23. Use of at least one iron complexing protein and at least one precursor of the metabolism of nitrogen monoxide and/or at least one chemical donor of nitrogen monoxide and optionally at least one iron salt, for preparing a nutritional supplement.
